(19) Europäisches Patentamt
European Patent Office
Office européen des brevets

(11) Publication number : 0 390 910 B1

(12) EUROPEAN PATENT SPECIFICATION

(45) Date of publication of patent specification : 13.12.95 Bulletin 95/50

(51) Int. Cl.$^6$ : G01N 33/53

(21) Application number : 89911926.7

(22) Date of filing : 10.10.89

(86) International application number : PCT/US89/04320

(87) International publication number : WO 90/04786 03.05.90 Gazette 90/10

# (54) HAPTEN DERIVATIZED CAPTURE MEMBRAN AND ASSAYS USING SUCH MEMBRANE

(30) Priority : 17.10.88 US 258894

(43) Date of publication of application : 10.10.90 Bulletin 90/41

(45) Publication of the grant of the patent : 13.12.95 Bulletin 95/50

(84) Designated Contracting States : AT BE CH DE FR GB IT LI LU NL SE

(56) References cited :
EP-A- 0 201 079
WO-A-85/05451
DE-A- 3 705 686
FR-A- 2 601 455
GB-A- 2 199 327
PATENT ABSTRACTS OF JAPAN, vol. 4, no. 144 (C 27), 14 July 1980

(73) Proprietor : MOLECULAR DEVICES CORPORATION
Menlo Oaks Corporate Centre
4700 Bohannon Drive
Menlo Park, CA 94025 (US)

(72) Inventor : OLSON, John, D.
1055 Lois Avenue
Sunnyvale, CA 94087 (US)
Inventor : ZUK, Robert, F.
1536 Los Montes
Burlingame, CA 94010 (US)
Inventor : ARMENTA, Richard, D.
1085 Ticonderoga Drive
Sunnyvale, CA 94087 (US)
Inventor : BURKE, Charles, R.
945 Old Trace Road
Palo Alto, CA 94306 (US)
Inventor : KUNG, Viola, T.
1055 Lemon Street
Menlo Park, CA 94025 (US)
Inventor : SHELDON, Edward, L., III
788 17th Avenue
Menlo Park, CA 94025 (US)

(74) Representative : Pett, Christopher Phineas et al
Frank B.Dehn & Co.
Imperial House
15-19 Kingsway
London WC2B 6UZ (GB)

EP 0 390 910 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Field of the Invention

This invention relates to a diagnostic reagents, capture membranes, which are useful in removing specifically binding complexes from solutions and assay methods using such reagents. The specifically binding complexes include antigen/antibody complexes, DNA, anti-DNA (antibody to DNA) or Single Strand DNA Binding Protein (such as SSB from E. coli), DNA, DNA hybrids, RNA, RNA hybrids, and similar specifically binding complexes.

### The Related Art

There are extensive teachings in the art of diagnostic assays and reagents involving specifically binding complexes. Antigen/antibody reactions are widely used to determine antigen and antibodies. The labeling of members of these complexes with detectable markers such as enzymes or florescent dyes is well known. The binding of antigen or antibodies and solid supports as a means of removing complexes from solutions is also known. The use of haptens such as biotin and anti-haptens such as streptavidin in diagnostic assay is extensively discussed in a review article that appears in Analytical Biochemistry 171, 1-32 (1988).

Biotin attached to a solid-support is described in U.S. Patents 4,282,287, 4,478,914, and 4,656,202. These patents describe precise layering technique wherein biotin is first attached to a solid-surface and the subsequent application of successive layers of avidin and extender results in a controlled modification of surface characteristics.

European Patent No. 87,307,850.5 (EP-A-0 259 186) is directed toward a method for routine plant-virus diagnosis which includes biotin attached to a macro-molecule that is conjugated to a sample of probe DNA. The probe containing compound is applied to a solid-matrix which has a test sample of DNA derived from plant tissue immobilized thereon. The presence of the target sequence is determined by washing the matrix with enzyme linked avidin followed by assaying for enzyme activity associated with the matrix.

U.S. Patent No. 4,467,031 describes an enzyme-immunoassay which utilizes the biotin-avidin system as a convenient and stable linking group for connecting a reporter enzyme to an antibody.

U.S. Patent No. 4,228,237 describes the use of the biotin-avidin system in a method for detection and determination of ligands. A surface having an antibody for the ligand of interest attached thereto is reacted with a sample of the ligand followed by a second ligand specific antibody that is conjugated with biotin. This complex is then reacted with an avidin conjugated enzyme and the results are determined by measurement of enzyme activity.

U.S. Patent No. 4,656,025 describes a screening assay for tumor globulin. A tumor globulin-biotin conjugate on ELISA plates is reacted with avidin conjugated enzyme and quantification of the tumor globulin bound to the plate is determined by the application of the appropriate chromogenic substrate thereto.

U.S. Patent No. 4,535,057 describes an immunoassay having biotin conjugated to a solid support through an antibody-virus complex. This biotin-antibody virus complex is then reacted with avidin conjugated to a reporter group or a label and the presence of the label associated with the surface is indicative of the presence of virus in the sample.

U.S. Patents 4,727,019, and 4,632,901 describe an immunoassay Wherein avidin is attached to a solid support and binds a ligand present in the sample to the support. U.S. Patent No. 4,298,685 describes a diagnostic reagent that also involves avidin immobilized on a solid support. U.S. Patent No. 4,582,810 describes a detection system wherein a suspension of particles having avidin covalently bound thereto reacts with a biotin-antibody complex to form a complex which results in a flocculent appearing solution.

GB-A-2199327 describes a porous membrane on which one partner of a binding pair is immobilised by means of a chemical activating agent. WO85/05451 describes a porous membrane to which one partner of a binding pair is bound or fixed. Both membranes may be used to entrap the other partner of the binding pair.

U.S. Patent No. 4,550,075 describes a method for ligand determination based on the biotin-avidin system without any solid support. EP-A-201079 describes a solid phase immobilised assay based on biotin-avidin binding.

U.S. Patent No. 4,486,530 describes an immunometric assay process that comprises a ternary complex of an antigenic substance, and a first and second antibody bound to the antigen in which the complex is removed from solution by filtering through a membrane.

Clinical Chemistry, 34, No. 8, p. 1585 (1988) describes a monoclonal antibody based noncompetitive avidin-biotin assay for luteinizing hormone (LH) in urine.

U.S. Patent No. 4,778,751, describes a method for measuring antigens which comprises: forming in a liquid

phase reaction a soluble complex wherein an antigen ($Ag_1$), antibody ($Ag_1$) or hapten (H) is linked through, respectively, a specific antibody (Ab), antigen (Ag) or anti-hapten (Anti-H), to a matrix which is soluble in the liquid phase and carries a ligand (X), the matrix capable of being chemically attached to more than one specific antibody (Ab), antigen (Ag) or anti-hapten (Anti-H); forming an insolubilized complex comprising a solid support linked to the ligand (X) of the soluble complex through an anti-ligand (Y), the insolubilized complex carrying a label (Z) linked to the antigen ($Ag_1$) through an antiantigen (Anti-$Ag_1$), to the antibody ($Ab_1$) through an anti-antibody (Anti-$Ab_1$) or to the hapten (H); washing the insolubilized complex; and observing the washed insolubilized complex for the presence of the label (Z) wherein the presence of the label (Z) is an indication of the level of the antigen ($Ag_1$), antibody ($Ab_1$) or hapten (H) in the sample.

European Patent Application No. 86111379.3 (EP-A-0 212 603) describes multilayer immunoassay test devices involving the use of labeled reagents comprising a chemical group having a detectable physical property such as fluorescence or color.

European Patent Application No. 88.308164.8 (EP-A-0 310 251) describes a method for determination of single-stranded DNA based on the binding of a single-stranded DNA to a single-stranded DNA-binding protein to which is bound a solid support.

Molecular Immunology, 34: 221-230 (1989) describes an ELISA system involving immobilization of biotinylated CAbs through linkage by streptavidin to biotinylated carrier proteins absorbed on polystyrene. The present invention provides technology for removal of specifically binding complexes from a solution and differs from the prior art in that the reagent of this invention is a porous membrane with a hapten preferably biotin bound directly or indirectly to the membrane.

## SUMMARY OF THE INVENTION

The present invention encompasses a capture membrane comprising a porous filter membrane having a hapten bound indirectly to the membrane via a macromolecule adhered to the membrane and covalently bound to the hapten.

## DETAILED DESCRIPTION OF THE INVENTION

As noted above, the present invention comprises a porous membrane or filter to which is bound a hapten; this membrane or filter being capable of filtering a solution that may contain a specifically binding complex having an anti-hapten bound to a binding member of the specifically binding complex.

The material of the membrane or filter is selected from material to which protein or other macromolecule can be adhered. A variety of materials may be used. Those skilled in the art will appreciate that porous membranes made of nylon, cellulose acetate, polyolefin, polyacrylamide, nitrocellulose or other porous materials may be employed in the present invention. Other synthetic or naturally occurring materials which will adhere a protein or other macromolecule may also be used. A preferred membrane is made from nitrocellulose.

Haptens are substances which do not elicit antibody formation unless complexed to macromolecules and which may be employed in the present invention as specific organic materials for which specific binding substances can be provided. Antibodies to haptens can be formed by binding the hapten to a protein to elicit an antibody response. A specific binding substance is any substance or group of substances having a specific binding affinity for the hapten to the exclusion of other substances. The employed hapten must be able to bind to a protein or other macro-molecule directly or through an extended linking group. Examples of haptens which may be used according to the instant invention include steroids such as estrone, estradiol, testosterone, pregnanediol and progesterone; vitamins such as $B_{12}$, biotin and folic acid; triiodothyronine, thyroxine, histamine, serotonin, digoxin, prostaglandins, adrenalin, noradrenalin, morphine, vegetable hormones and antibiotics such as penicillin.

When the hapten is a substance having a naturally occurring receptor, the receptor can be utilized as the anti-hapten provided the receptor can be isolated in a form specific for the hapten: Illustrative haptens which have naturally occurring receptors include thyroxine, many steroids, polypeptides, such as insulin, angiotensin, biotin and many others. Receptors for this class of haptens are usually proteins or nucleic acids.

Extended linking groups are groups that will bind the hapten to the protein or macro-molecule in such a way that the hapten has better access to the anti-hapten. Extended linking groups useful in the present invention include succinylated polylysine, dextran, polyethylene glycol, and preferentially a polyamido ether extending group. These extended linking groups may be used separately or in combination to obtain extended linking groups of varying lengths and binding properties. Extended linking groups are preferred for use with serum samples especially lipemic serum samples. Evidently, there are interfering substances in serum samples, the interference from which is overcome by the extended linking group. Where an extended linking group is not

needed, a hapten such as biotin, without an extended binding group is bound to a functional group on a membrane or to a function group on a protein which can be disbursed on the membrane.

The extended linking group must be able to bind to the protein or macromolecule. Preferentially the extended linking group having an hapten bound to one end will be bound to the protein or macro-molecule with an amide bond; the amine of the amide bond arising from the protein and the carboxyl of the amide bond arising from the carboxy terminus of the extender group. Free carboxyl or hydroxyl groups on proteins can likewise be used.

The proteins and macro-molecule of the present invention include, but are not limited to, bovine serum albumin (BSA), bovine gamma globulin, and fibrinogen.

The complex produced by specific binding which is removed from the solution as it passes through the filter comprises two binding members and an antigen, with the proviso that one binding member is bound to an anti-hapten and the other binding member is bound to a labelling group.

The anti-haptens bound to the binding members of the present invention comprise the molecules described above that act as receptors to the above mentioned haptens. Antibodies are preferred binding members which are conveniently labeled with enzymes or fluorescent dyes and are also conveniently bound to anti-haptens such as avidin or streptavidin or antibodies to haptens.

This embodiment of the invention can be viewed as follows:

| Capture Membrane | | Complex |
|---|---|---|
| membrane - hapten | . | anti hapten - Ab Ag Ab-(labeled) |
| membrane - (biotin) | . | (streptavidin)-Ab' Ag' Ab(enzyme) |

Persons having skill in the art will recognize that capture membranes may also be employed in sequential assays. In such an assay, a series of filtration steps is used to capture and detect a substance to be determined. Such an assay may have many advantages over standard, non-sequential assays.

For example, a solution of anti-hapten could be filtered through a haptenated porous filter membrane, whereby the anti-hapten would be captured on the membrane. A solution containing an anti-hapten binding substance would subsequently be filtered through the membrane, thereby capturing the anti-hapten binding substance. This anti-hapten binding substance may be a hapten or other substance which binds to an anti-hapten. The anti-hapten binding substance preferably is modified with binding sites for a substance to be determined. Filtering a solution of a substance to be determined through the membrane will capture the substance to be determined on the membrane. The substance to be determined may then be detected on the membrane by filtering and labeling the substance to be determined with a detectable label and detecting the label on the membrane. The label may then be detected by various methods in the art. For example, an electrode which is a semiconductor may be employed in one embodiment of the present invention.

Persons having skill in the art will recognize that many combinations of sequential assays may be performed. Complexes may be formed between anti-haptens, haptens, antigens, antibodies, substances to be determined and detectable labels. These complexes may have one or more components. Thus, assays may be designed to avoid interference from specific substances.

This embodiment of the invention can be viewed as follows:

| Capture Membrane | | Complex |
|---|---|---|
| membrane-hapten-antihapten | . | hapten-Ab Ag Ab-(labeled) |
| membrane-(biotin) (Streptavidin) | . | biotin-Ab' Ag' Ab-(enzyme) |

Antibodies may be determined in a manner similar to that for an antigen. Complexes may be formed between an anti-hapten, an antibody, an antigen bound to a hapten or anti-hapten and another antigen bound to a labeling group.

The haptens and anti-haptens comprise those molecules described above as haptens and anti-haptens. Antigens may be conveniently labeled with enzymes or fluorescent dyes and are also conveniently bound to haptens or anti-haptens.

These embodiments of the invention may be viewed as follows:

| Capture Membrane | | Complex |
|---|---|---|
| membrane-hapten | . | anti-hapten hapten-Ag Ab Ag-labeled |
| membrane-(biotin) | . | (streptavidin)(biotin)-Ag Ab Ag-(enzyme) |

The antibodies employed in the present invention may be either polyclonal or monoclonal antibodies and are produced in response to the target antigen of the assay. Methods for the production of antibodies to various biological substances are well known in the art.

The antigens targeted by the assay include, but are not limited to antigens such as IgE, prostatic acid phosphatase, prostate specific antigen, alphafetoprotein, carcinoembryonic antigen, luteinizing hormone, creatine kinase MB, Human Chorionic Gonadotropin (HCG) and other antigens in serum, plasma, urine, or other liquid media.

Polydeoxyribonucleotides can be determined by reactions with single strand DNA binding protein (SSB) and anti-DNA antibodies. Thus, various combinations of labeled SSB or anti-DNA and biotinylated SSB or anti-DNA are employed. In this embodiment streptavidin is bound to the biotinylated SSB or anti-DNA so that the complex can be bound to the capture membrane having biotin. In place of SSB, an oligonucleotide probe may be used to detect DNA. The article in Biochemistry, 25:21 (1986) describes the large scale over production of single-strand binding protein (SSB) from E. coli. Monoclonal antibodies to DNA have been used to measure DNA in biological fluids, Journal of Immunological Methods, 88, (1986) 185-192.

These embodiments of the invention can be viewed as follows:

| Capture Membrane | | Complex |
|---|---|---|
| membrane-biotin | . | streptavidin-biotin-anti-DNA/ DNA/SSB-enzyme |
| membrane-biotin | . | streptavidin-biotin-SSB/DNA/ anti-DNA-enzyme |
| membrane-biotin | . | streptavidin biotin-oligonucleotide probe/DNA/ oligonucleotide probe-enzyme |

Labeling groups that may be employed in the present invention include enzymes, fluorescent labels and radionuclides. The preferred label is an enzyme that is linked to the antibody at a position which does not interfere with the binding of the antibody to the antigen. Thus, the enzyme should possess potentially reactive groups to which the antibody can be coupled without destroying enzyme activity and should not occur naturally to an appreciable extent in the liquid to be assayed for the specific biological substance. In addition, the enzyme should have a relatively long shelf life, a high specific activity and also be capable of being easily assayed, for example, with a visible light spectrophotometer.

Examples of enzymes which may conveniently be employed in the process of the present invention are, malate dehydrogenase, lipase, delta-5-ketosteroid isomerase, yeast alcohol dehydrogenase, yeast glucose-6-phosphate dehydrogenase, alpha glycerophosphate dehydrogenase, triose phosphate isomerase, horseradish peroxidase, alkaline phosphatase, asparaginase, glucose oxidase, beta-galactosidase, and more preferably, urease. Normally, it is preferred that the enzyme be in a pure form, free of contaminating proteins.

The preparation of the enzyme-labelled biological substances for use in the present invention can be accomplished in various ways known in the art. Examples of the coupling of biological substances to enzymes are described in, for example, L. A. Steinberger, Immunocytochemistry, Prentice Hall, New Jersey (1974).

Although a radionuclide such as $^{125}I$ or $^{32}P$ may also be used as the label non-radioactive labels are preferred.

After filtering the solution suspected of containing the antigen in a specifically binding complex through the porous filter membrane, the presence of labelled antibody on the porous membrane is then determined as an indication of the presence of the target antigen in the sample. In the case of an enzyme label this may be done by addition of a solution of a color forming substrate to the porous member to allow the substrate to react with the enzyme. Determinations can conveniently be made by the devices and methods described in U.S. Patents 4,591,550, 4,704,353, and EP-A-0 255 223 and assigned to the same assignee as this application.

The invention is illustrated further by the following examples.

## BRIEF DESCRIPTION OF THE DRAWINGS

FIG. 1 illustrates the improved efficiency of the biotin membrane (BXL-BSA, biotinylated-Bovine Serum Albumin) versus the avidin (Streptavidin) membrane.

FIG. 2 illustrates the efficiency of capture of streptavidin at various densities of biotin using two different types of membrane and two different flow rates.

FIG. 3 illustrates the capture efficiency of 0.8μ nitrocellulose filter at various concentrations of BXL-BSA on the membrane and at various Streptavidin flow rates.

FIG. 4 illustrates the determination of TSH.

FIG. 5 is a standard curve generated by the dual probe assay.

FIG. 6 is a semilog plot illustrating amounts of polymerase chain reaction (PCR) product determined by the dual probe assay.

FIG. 7 is a graph of the efficiency of the PCR determined using the dual probe assay.

FIG. 8 is standard curves for the determination of MuIgG by sequential and simultaneous procedures.

## DESCRIPTION OF THE PREFERRED EMBODIMENTS

A preferred capture membrane is made according to the following reaction scheme.

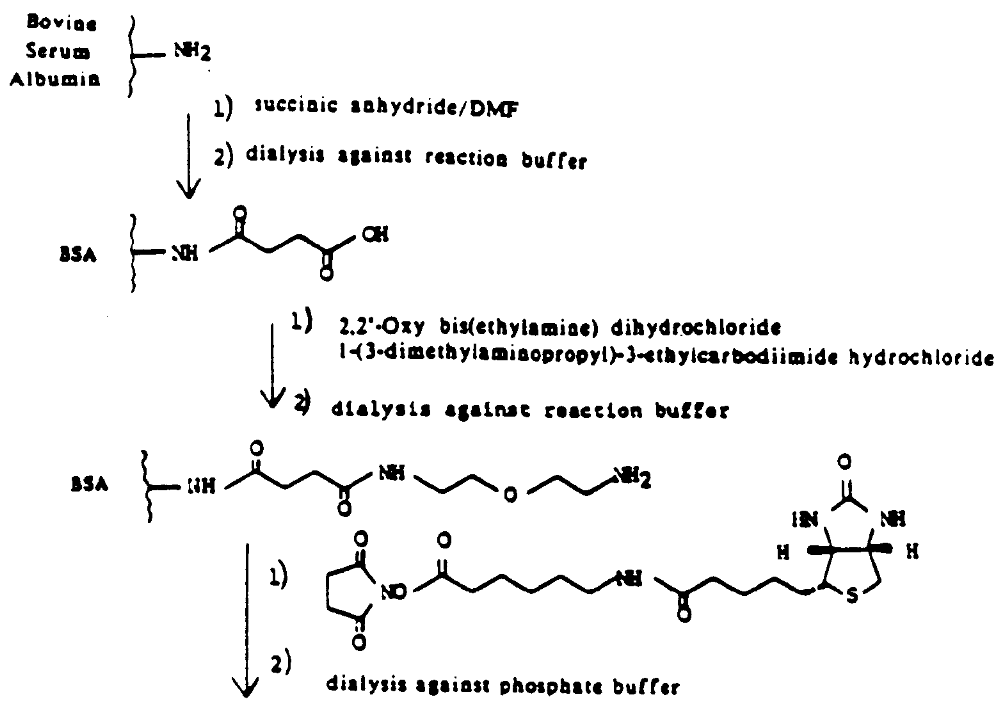


A compound of the formula:

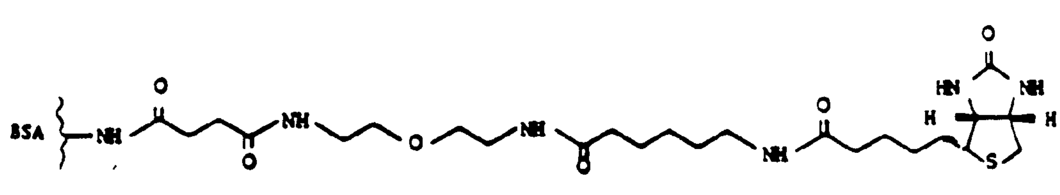

wherein 5-20 biotins with the extended linking group are bound to BSA.

## Example 1

### Preparation Of Capture Membrane

A pH 8.2 reaction buffer was prepared by dissolving 8.40g sodium bicarbonate and 8.76g sodium chloride in sufficient distilled water to make 1L of solution.

10.00g Bovine Serum Albumin (BSA) was dissolved in 250mL of pH 8.2 reaction buffer and the resulting BSA solution dialyzed exhaustively against reaction buffer with a dialysis filter. The volume after dialysis was still 250mL.

The BSA concentration, as determined by absorbance at 280nm at this point was 40mg/mL. A TNBS assay performed on this BSA solution indicated 18 free amino groups per BSA. TNBS is a 2,4,6-trinitrobenzene sulfonic acid acid/sulfite based assay for free amino groups in a protein which is a modification of the procedure described by Palmer et al., Clin. Chem., 15: 891-901, 1969. An aliquot was removed for later TNBS assays. To the BSA solution was then added 6.32g of $K_2CO_3$ and the resulting solution was stirred vigorously as a solution of 3.34g succinate anhydride in 83.5mL DMF was added slowly over 14 minutes. The resulting solution was then stirred for an additional 5 minutes and allowed to stand at room temperature for one hour. The solution was then exhaustively dialyzed against reaction buffer and concentrated to 250mL using a Minitan concentrator.

The BSA concentration at this point was determined by absorbance to be 40mg/mL. A TNBS assay performed on the succinylated-BSA solution indicated less than one free amino group per succinylated-BSA.

At this point, 25mL of a 1M solution of 2,2′-Oxy bis(ethylamine) dihydrochloride (pH 8.2) in reaction buffer was added to the stirred BSA solution. Next, 6.39 g of 1-(3-dimethylaminopropyl)-3-ethylcarbodiimide hydrochloride was added to the vigorously stirred BSA solution. Stirring was stopped after all of the carbodiimide had dissolved and the resulting solution covered and incubated at room temperature for one hour.

The solution was then exhaustively dialyzed against reaction buffer and concentrated to 250mL using a Minitan® (Millipore) concentrator.

The BSA concentration was again determined by absorbance to be 40mg/mL. A TNBS assay run on the above prepared BSA-Oxy bis(ethylamine) solution indicated the presence of 15 free amino groups. A TNBS assay performed on the final dialysis buffer indicated sufficient dialysis of free amines from the BSA solution.

To the BSA solution was then added 1.51g succinimidyl 6-(biotinamido) hexanoate and the solution was stirred vigorously for 5 minutes after which it was covered and incubated for one hour at room temperature.

The biotinylated-BSA solution was exhaustively dialyzed against a pH 7 phosphate buffer. The phosphate buffer was prepared as follows: 4.97g of dibasic sodium phosphate, 2.07g of monobasic sodium phosphate and 6.77g of sodium chloride were dissolved in sufficient distilled water to make 1L of solution.

The concentration of biotinylated-BSA in solution after dialysis was determined by absorbance to be 15mg/mL.

A TNBS assay performed on the biotinylated-BSA solution indicated the presence of three free amino groups. The difference between the three free amino groups on the biotinylated-BSA and 15 on the BSA - Oxy bis(ethylamine) is 12. This is the number of biotin molecules in the biotinylated-BSA.

0.8μ nitrocellulose membrane or Immobilon membrane was cut into sheets measuring 20cm by 30cm. 100mL biotin immobilizing solution having 5mg biotinylated BSA/mL phosphate buffer was placed in a trough.

The membrane is immersed into the protein solution. The wetted membrane was placed on the bottom of a glass dish, the dish inverted and the membrane incubated for 45 minutes. 500mL of phosphate buffer was added to the dish and incubated for 15 minutes. This solution was then decanted.

A 0.1% glutaraldehyde fixing solution was prepared by adding sufficient phosphate buffer to 4mL of a 25% glutaraldehyde solution to make 1L. 500mL of this glutaraldehyde fixing solution was then added to the dish containing the nitrocellulose membrane, and the contents were incubated at room temperature for two hours. A solution of 0.1 M ethanolamine pH 9.5 was added to a dish containing the Immobolin membrane and the contents were incubated at room temperature overnight.

After decanting the fixing solution or the ethanolamine, 500mL phosphate buffer was added and the contents incubated for 15 minutes. The solution was again decanted, 500mL distilled water added and the contents incubated for 15 minutes.

The water incubation was repeated once. The membrane was then inserted between two sheets of Whatman 3MM blotting paper to remove excess liquid and upon removal from the blotting paper was placed between fiberglass screen material. The screen holding the membrane was placed into a convection oven at 65°C for 15 minutes.

The membrane was removed from the oven and placed into a vacuum desiccator at room temperature for storage.

**Analysis of the Capture Efficiency of Membrane Coated with Biotin**

The characteristics of binding at various flow rates of streptavidin to a biotin coated membrane (BXL-BSA) and of biotinylated BSA to a streptavidin coated membrane is shown in Figure 1. Nitrocellulose membrane was coated with either streptavidin or BXL-BSA so as to allow the binding of approximately equivalent amounts of its corresponding binding partner. Three hundred uL of radiolabled streptavidin or BXL BSA was filtered through the appropriate membrane at various flow rates. The radioactivity captured on each membrane at the slowest flow rate was assigned 100% capture. The percentage of counts captured is plotted as a percentage of maximum capture against the log of the flow rate.

It is possible that the maximum flow rate tested was still too fast for the streptavidin membrane to capture 100% of the BXL-BSA since the capture curve had not begun to level off. However the biotin coated membrane was able to capture 95% of the streptavidin with a flow rate of 75µL/minute whereas for the streptavidin membrane a flow rate of 17µL/minute was needed to capture the same percentage of BXL-BSA.

The effect of biotin density (determined by the biotin/BSA ratio referred to as hapten number) on the capture of streptavidin is shown in Figure 2. Two types of membranes were tested at various flow rates: 0.45 nitrocellulose [NC] and 0.65µ Immobilon [IM] membrane. The latter, manufactured by Millipore Laboratories is a polyvinylidene difluoride based membrane which is coated with a hydrophilic polymer and is activated to allow covalent protein attachment. The membranes were coated with bovine serum albumin (BSA) which was labeled to various extent with biotin. The NC and IM membranes contained approximately 10 $\mu g/cm^2$ and 2$\mu g/cm^2$ of BSA respectively. The numbers in parenthesis refer to the time taken for 300µL of $^{125}$I-streptavidin to flow through the membrane. The figure shows that the higher the biotin density on the BSA the better the capture of streptavidin on the membrane. This is more evident on the IM membrane. The greater effect on the IM membrane is due to the lower amount of protein on the membrane. This can be concluded from the next figure (Fig. 3).

The effect of the protein loading on the ability of nitrocellulose membrane coated with biotin-BSA to capture streptavidin is shown in Figure 3. Biotin-BSA, with a hapten number of 10.3, was immobilized onto nitrocellulose membrane at various concentrations. The X axis indicates the concentration of biotin-BSA employed to coat the membrane. The amount of biotin-BSA on the membrane is approximately 2.5, 5, 10, 30, 50 $\mu g/cm^2$. Three hundred µL of $^{125}$I-streptavidin was filtered through the membrane in 1.5 minutes and 5 minutes. The Y-axis represents the counts captured by the membrane as a percentage of the maximum counts bounds. As can be seen in the figure, the amount of biotin-BSA on the membrane also plays a role in the capture efficiency of streptavidin on the membranae.

The preceding figures demonstrate several key aspects of the invention: (1) Given equal binding capacities and at a given flow rate, the biotin coated membrane is more efficient at capturing streptavidin than the streptavidin coated membrane is at capturing biotin labeled protein. (2) Capture efficiency of the biotin membrane is determined by the biotin density on the membrane. The biotin density is controlled by both the hapten number on the BSA and by the amount of biotin-BSA on the membrane.

**Use of Biotin Membrane in Assay for TSH**

A pH 7.0 wash buffer was prepared to contain 100mM sodium phosphate, 150mM NaCl, 0.1% bovine gamma globulin and 0.05% of a surfactant such as polyoxyethylene sorbitan monooleate.

A nitrocellulose membrane was coated with biotinylated BSA (BXL-BSA) as described above. One square centimeter of the biotinylated membrane was mounted with transfer tape over a 6mm diameter hole of a 1cm x 10cm vinyl acetate stick.

The stick containing the membrane was placed into a filter unit, the upper portion of which is a funnel shaped cartridge having a 4mm diameter opening that forces liquid to flow through the membrane.

A pH 7.4 conjugate-sample dilution buffer was prepared to contain 10mM sodium phosphate, 150mM NaCl, 10mM $Na_2SO_3$, 1mM 2-mercaptoethanol, 0.01% $NaN_3$, 0.1% bovine gamma globulin, 0.05% of a surfactant such as polyoxyethylene mono-oleate, 0.25% octoxynol, 1mM EDTA. 2µg/mL streptavidin - anti-thyroid stimulating hormone and 2µg/mL urease - anti-thyroid stimulating hormone were added to the buffer solution immediately prior to running the assay.

750µL conjugate-sample dilution buffer was added to 250µL of serum and the resulting mixture incubated in a covered plastic tube for 1 hour at 37°C.

450µL of this sample-conjugate mixture was filtered under vacuum via syringe pump through the mounted biotinylated membrane in three minutes.

The membrane was washed by filtering 500µL of the pH 7.0 wash buffer through it in approximately 1 minute.

The stick was then removed from the filter unit and inserted into a sensor assembly having a silicon wafer. See the device of U.S. Patent 4,591,550. The filtration area of the stick matched the light interrogated portion of the silicon wafer. Only one site of the reader was used and the plunger position was set at 70μ from the silicon wafer surface. The reaction was monitored for 150 seconds for low signals (250μV/sec) and for 50 seconds for higher signals. Figure 4 illustrates the typical results for TSH determination.

## Example 2

### The Effect of Biotin Attachment Arm On The Recovery of Spiked TSH From Pooled Sera

| Capture Ligand Recovery | Sample | Rate | % |
|---|---|---|---|
| Biotin-BSA | Buffer | 1733 | "100" |
| Biotin-BSA | Sera | 191 | 11 |
| BXL-BSA | Buffer | 2592 | "100" |
| BXL-BSA | Sera | 2731 | 105 |

The table above indicates improvement in recovery of TSH from serum when the long chain form of biotin-BSA (BXL-BSA) is used compared with short-chain biotin-BSA (Biotin-BSA). The above results were obtained with the standard assay protocol described above. Either 1mL pooled sera (sera) of conjugate-sample dilution buffer (buffer) was spiked with 1ng of TSH. Samples were normalized to the rates of the buffer samples (% recovery) for each type of membrane. In addition to the greater recovery in serum, the higher signal in buffer indicates a greater overall capture efficiency of the conjugate complex with BXL-BSA than with short chain biotin.

## Example 3

### DNA Assay Dose-Response

Membrane: biotin-BSA coated nitrocellulose membrane (0.8μ pore size).
DNA sample: 0, 5, 10, 25, 50 pg of single-stranded Calf thymus DNA in 200μl of phosphate buffered saline (PBS).
Reagent: 1μg/ml Streptavidin, 1ng/ml SSB-urease, 10ng/ml biotin-anti-DNA, 1% BSA in 10mM Tris·HCl buffer, 1mM EDTA (pH 7.4).
Assay Protocol: 200μl of DNA sample was incubated with 500μl of reagent at 37° for 30 minutes. The mixture was filtered through the biotin-BSA coated membrane at a flow rate of about 100μl/min. The membrane was then washed with 1cc of pH 5 wash buffer (1mM sodium acetate, 0.1M NaCl, 0.05% polyoxyethylene monooleate) at a maximum flow rate of about 6ml/min). After washing, the membrane was transferred to pH sensor chambers (U.S. Patent 4,591,550), containing the substrate (pH 5 wash plus 100mM urea) and the pH response was read.

#### Results

| DNA (Pg/sample) (μV/sec) | Rate of Signal |
|---|---|
| 0 | 62 ± 3 |
| 5 | 106 ± 7 |
| 10 | 156 ± 10 |
| 25 | 310 ± 1 |
| 50 | 778 ± 70 |

## Example 4

### Detection Of DNA In Insulin And Granulocyte Macrophage Colony Stimulating Factor (GMCSF) Samples

Membrane: biotin-bSA coated nitrocellulose membrane (0.8μ pore size).
Pre-treatment of Porcine Insulin: 10mg/mL insulin (in 10mM tris buffer, 1mM EDTA, pH 8.5) was digested with 100μg/mL of proteinase K at 55°, overnight. 0, 5, and 50 pg of calf thymus DNA were spiked into 1mg of insulin digest. In another set of experiments, 0, 5, and 50pg of calf-thymus DNA were spiked into PBS solution containing 0.4mg of GMCSF (total volume was 200μl). All samples were heated at 100°C for 5 minutes to inactivate proteinase K and denature DNA to single-stranded. Insulin samples were cooled on ice and GMCSF samples were cooled down to room temperature.

200μl of sample was mixed with 500μl of reagent (200ng/ml streptavidin, 2.5ng/ml biotin-SSB, 9 ng/ml urease-anti-DNA; in 1% BSA, 0.25% octoxynol, 10mM Tris·HCl buffer, 1mM EDTA, pH 7.5).

The samples were incubated at 37° for 30 minutes and filtered through the membrane. 1cc of wash solution 5mM sodium phosphate, pH 7, 0.1M NaCl 0.05% polyoxyethylene sorbitan mono-oleate) was filtered through the membrane twice. The signal was read with a pH sensor of the type described in U.S. Patent 4,591,550.

Results

| Sample | Rate (μV/sec) |
|---|---|
| insulin | 39 |
| insulin + 5 pg DNA | 56 |
| insulin + 50 pg DNA | 254 |
| GMCSF | 21 |
| GMCSF + 5 pg DNA | 40 |
| GMCSF + 50 pg DNA | 192 |

## EXAMPLE 5

### Dual Probe Assay

Dual probe assay mixtures were prepared to contain 1:2 serial dilutions of Hinf1 digested pGEM3 starting at 25 ng (7.8 x $10^9$ target molecules) per assay mixture, 2 ng of 5′ biotinylated 20mer (CCAGTTACCTTCGGAAAAAG), and 0.5 ng of 5′ fluoresceinated 20mer (TAGCTCTTGATCCGGCAAAC) in 100 μl of 3X PBSE (a pH 7.4 solution containing 30 mM sodium phosphate, 450 mM NaCl, and 3 mM EDTA) having 0.25% of a surfactant such as polyoxyethylene sorbitan mono-oleate. This sequence of each 20-mer is also found on the same HinfI fragment of pGEM3. The sample DNA was then denatured by heating the mixture to 100°C for 5 minutes.

The mixture was subsequently incubated at 50 to 55°C for 30 minutes. To the mixture was then added 1 ml of a solution prepared to contain 1 μg/ml streptavidin, 8 μg/ml anti-fluorescein-urease conjugate in 3X PBSE, 0.25% polyoxyethylene sorbitan mono-oleate, 1 mg/ml BSA and 80 μM N-acetylcysteine. This resulting conjugate solution was incubated for 30 minutes at room temperature.

The incubated conjugate solution was then filtered through a biotin coated nitrocellulose membrane mounted on a plastic stick. Next, 1 ml of threshold wash (10 mM sodium phosphate, 100 mM NaCl, 0.05% polyoxyethylene sorbitan mono-oleate, and 0.05% $NaN_3$, pH 6.5) was filtered through the membrane after the conjugate solution and the membrane was dipped into 50 ml of threshold wash. The stick was then inserted into a pH sensor chamber of the type described in U.S. Patent No. 4,591,550 containing substrate solution (100 mM urea in threshold wash). The pH response was then read yielding the results displayed in Fig. 5.

## EXAMPLE 6

### Application of Dual Probe Assay to Polymerase Chain Reaction (PCR)

1. Amplification of 0.1 kbp region in pGEM3 using PCR

A polymerase chain reaction (PCR) mixture was prepared to contain 100 mM Tris-HC1 (pH 8.3), 10 mM MgC13, 1 mM each dNTP, 1 μM primer 1(CAAAAAAACCACCGGTACCAG) , 1 μM primer 2 (AGTATTTGG-TATCTGCGCTCTG), 10 pg pGEM3 (3.15 x $10^6$ target molecules) previously digested with Hinf1. The region to be amplified contains each of the 20-mer sequences described in Example 5. This PCR mixture was incubated at 100°C for 5 minutes.

Immediately after incubation, 2μl of 5U/μl Taq Polymerase (Perkin Elmer) was added to the PCR mixture which was thoroughly mixed and then incubated at 94°C.

Using an Eppendorf thermal cycler, the incubation temperature was cycled between 94°C and 55°C. The thermal cycler was set so that each cycle lasted about 3 minutes. PCR samples were removed from the mixture in the cycler at cycles 13, 15 and 17 when the temperature reached 56°C. These samples were stored at -20°C for later use in the dual probe assay. Blank samples which did not contain pGEM3 DNA were prepared similarly for use as a negative control.

2. Dual Probe Assay using PCR Sample

10 μl of a PCR sample prepared above was combined with 90 μl of the dual probe assay mixture prepared in Example 5 and the entire 100 μl mixture was denatured at 100°C for 5 minutes.

The mixture was subsequently incubated at 50 to 55°C for 30 minutes. To the mixture was then added 1 ml of a solution prepared to contain 1μg/ml streptavidin, 8 μg/ml antifluorescein-urease conjugate in 3X PBSE, 0.25% polyoxyethylene sorbitan mono-oleate, 1 mg/ml BSA and 80 μM N-acetylcysteine. This resulting conjugate solution was incubated for 30 minutes at room temperature.

The incubated conjugate solution was then filtered through a biotin coated nitrocellulose membrane mounted on a plastic stick. Next, 1 ml of threshold wash (10 mM sodium phosphate, 100 mM NaCl, 0.05% polyoxyethylene sorbitan mono-oleate, and 0.05% $NaN_3$, pH 6.5) was filtered through the membrane after the conjugate solution. The stick mounted membrane was dipped into 50 ml of threshold wash and then inserted into a pH sensor chamber of the type described in U.S. Patent No. 4,591,550 containing substrate solution (100 mM urea in threshold wash). The pH response was then read yielding the results displayed in Figs. 6 and 7.

The specific signal (the difference between the signals from the samples with and without target DNA) increases more than linearly from cycle 13 to cycle 17 (Fig. 6). Figure 3 shows the amount of specific signal from cycles 13, 15, and 17 on a semilog plot. This graph suggests that the amount of specific signal and therefore PCR product, as determined by comparison with the amount of specific signal generated by known amounts of plasmid DNA (e.g., Fig. 5), increases exponentially from cycle 13 to cycle 17. The increase in specific signal per cycle fits a line that implies that the PCR amplification factor is 1.54 per cycle. Since the theoretical PCR factor is 2.00, the apparent efficiency is 77%.

The kind of measurements taken in this experiment have at least two potential uses. First, the determination of amplification factor is useful in experimental optimization of PCR conditions. For example, there is clearly room for further optimization in the experiment described above. Second, the exponential plot shown in Fig. 7 can be used to determine by extrapolation the amount of starting analyte. For example, the amount of nucleic acid from a pathogen, such as HIV, could be determined. This would be useful in assessing the response to a therapeutic regimen.

## EXAMPLE 7

### A. Simultaneous and Sequential Determination of Murine IgG

1. Simultaneous Determination Procedure

A mixture was prepared to contain MuIgG, biotinylated-anti-MuIgG, fluoresceinated-anti-MuIgG, streptavidin, and anti-fluorescein-urease. This resulting mixture was incubated for 1 hour at 37°C.

Assay buffer was added to the incubated mixture which was then filtered over a period of 15 minutes through a biotin coated nitrocellulose membrane mounted on a plastic stick. The membrane was washed with pH 7.0 phosphate wash buffer over 5 minutes. The stick as then inserted into a pH sensor chamber of the type

described in U.S. Patent No. 4,591,550 containing substrate solution (urea in threshold wash). The pH response was then read yielding the results shown in the following table and figure 8.

2. Sequential Determination Procedure

A mixture of MuIgG, biotinylated-anti-MuIgG and fluoresceinated-anti-MuIgG was prepared and incubated for 1 hour at 37°C. To this incubated mixture was then added streptavidin. The resulting mixture containing streptavidin was filtered over a period of 10 minutes through a biotin coated nitrocellulose membrane mounted on a plastic stick. The membrane was then washed with pH 7.0 phosphate wash buffer over 5 minutes.

A solution of anti-fluorescein-urease was next filtered over 5 minutes through the membrane which was subsequently washed over 5 minutes with pH 7.0 phosphate wash buffer. The stick was then inserted into a pH sensor chamber of the type described in U.S. Patent 4,591,550 containing substrate solution (urea in threshold wash). The pH response was then read yielding the results shown in the following table and figure 8.

**Determination of Murine IgG by**
**Simultaneous and Sequential Procedures**

Sequential

| pg MuIgG | μvolt/sec | S.D.(n=4) |
|---|---|---|
| 0 | 54 | 3.0 |
| 100 | 124 | 3.0 |
| 500 | 403 | 16 |
| 1000 | 758 | 93 |

Simultaneous

| pg MuIgG | μvolt/sec | S.D. (n-=4) |
|---|---|---|
| 0 | 120 | 11 |
| 100 | 209 | 11 |
| 500 | 658 | 24 |
| 1000 | 1247 | 112 |

**EXAMPLE 8**

**Simultaneous and Sequential Determinations of Anti-Human Chorionic Gonadotropin**

Human chorionic gonadotropin (HCG) was labeled with biotin to yield biotinylated HCG. Another sample of HCG was labeled with fluorescein to yield fluoresceinated HCG.

1. Sequential Determination of Anti-HCG

400μl samples were prepared to contain 0, 4, 20 and 100 ng of anti-HCG. Each sample was combined with 100μl of solution containing 10 ng of biotinylated HCG and 10 ng of fluoresceinated HCG and the resulting 500μl mixtures were incubated for 1 hour at room temperature.

To each incubated mixture was then added 500μl of a 4 ng/ml streptavidin solution and after mixing, were filtered through a biotin coated nitrocellulose membrane mounted on a plastic stick. Each membrane was then washed with 2 ml of pH 7.0 phosphate wash buffer. 500μl of a 4 μg/ml solution of urease-anti-fluorescein was filtered through the membrane which was again washed with 2 ml of the phosphate wash buffer.

The sticks were then inserted into a pH sensor chamber of the type described in U.S. Patent No. 4,591,550 containing urease substrate solution. The pH response was read generating the results displayed in the table

below.

2. Simultaneous Determination of Anti-HCG

100µl samples were prepared to contaon 0, 4, 20, and 100µl of anti-HCG. Each sample was mixed with 100µl of the biotinylated-HCG, fluoresceinated HCG solution used in part 1 of this example and with 100µl of the 4µg/ml urease-anti- fluorescein solution. The resulting mixture was incubated for 1 hour and 20 minutes at room temperature.

To each table was added 1 ml of assay buffer and the resulting mixture filtered through a biotin coated nitrocellulose membrane mounted on a plastic stick. The membrane was washed with 2 ml of wash buffer and read in a pH sensor as described above. The results are displayed below.

This sequential antibody assay may be used to measure an immune antibody response to a vaccine. The specific antibody may be detected at concentrations as low as 1%.

**Sequential and Simultaneous Determination of anti-HCG**

| | Rate (µv/sec) | |
|---|---|---|
| Anti-HCG (ng) | Sequential | Simultaneous |
| 0 | 115 ± 4 | 120 ± 15 |
| 4 | 130 ± 6 | 121 ± 2 |
| 20 | 187 ± 7 | 165 ± 7 |
| 100 | 500 ± 20 | 271 ± 17 |

**EXAMPLE 9**

**DNA Dose Response Assay-Alternate Configuration**

DNA samples were prepared to contain 0, 5, 10, 25, 50, 100, 150 and 200 pg of single-stranded DNA in 500µL of phosphate buffered saline. A dose-response reagent was prepared to contain 5pg/mL streptavidin, 2.25 ng/mL biotinylated single-stranded binding protein, and 93.8 ng/mL anti-DNA urease in Tris-EDTA formulation buffer. The 500 µL DNA samples were heat-denatured at 95°C for 10 minutes and subsequently cooled and combined with 1.0mL of dose-response reagent. These mixtures were incubated at 37°C for 1 hour and each was filtered through a biotin-BSA coated nitro-cellulose membrane mounted on a plastic stick. Each membrane was washed with pH 6.5, 10mm phosphate buffer and the stick inserted into a pH sensor chamber of the type described in U.S. Patent No. 4,591,550 containing urease-substrate. The pH response was read generating the data in the table below.

Results

| pg DNA | Rate (µvolt\sec, mean of 8 tests) | Standard Deviation | Coefficient of Variance (%) |
|---|---|---|---|
| 0 | 76.3 | 12.7 | 16.0 |
| 5 | 148 | 15.6 | 13.5 |
| 10 | 222 | 35.2 | 15.9 |
| 25 | 488 | 23.3 | 4.6 |
| 50 | 981 | 50.9 | 5.2 |
| 100 | 2048 | 61.3 | 3.0 |
| 150 | 3145 | 112.6 | 3.5 |
| 200 | 4662 | 189.5 | 4.1 |

## Claims

1. A capture membrane comprising a porous filter membrane having a hapten bound indirectly to the membrane via a macromolecule adhered to the membrane and covalently bound to the hapten.

2. A capture membrane according to Claim 1 wherein the hapten is covalently bound to the macromolecule via an extended linking group.

3. A capture membrane according to claim 1 or claim 2 wherein the macromolecule is a protein.

4. A capture membrane according to any one of claims 1 to 3 wherein the hapten is biotin and the anti-hapten is avidin or streptavidin.

5. A capture membrane according to claim 3 wherein the protein is bovine serum albumin, bovine gamma globulin or fibrinogen.

6. A capture membrane according to claim 4 or claim 5 wherein about 5 to 20 biotin molecules are bound to each bovine serum albumin molecule.

7. An assay where a specifically binding complex is removed from solution by a membrane which binds the specifically binding complex whereafter the said complex on said membrane is detected by means of a detectable label attached to said complex characterised in that the assay comprises the use of the porous filter membrane of any of claims 1 to 6 to capture said specific binding complex by reaction with an anti-hapten bound to a member of the specifically binding complex.

8. An assay as claimed in claim 7 which comprises the steps of:
   (a) forming in solution a specifically binding complex between binding members and the substance to be determined wherein one binding member has bound to it an anti-hapten and another binding member having a detectable label;
   (b) filtering the solution containing the specifically binding complex through the porous filter membrane of any of claims 1 to 6, whereby binding between the hapten and anti-hapten captures the specific binding complex on the membrane; and
   (c) detecting the label attached on the membrane.

9. An assay comprising the steps of:
   (a) forming in solution a specifically binding complex between the substance to be detected and binding members and anti-hapten wherein one binding member has a detectable label, and one binding member has bound to it a hapten;
   (b) filtering the solution containing the specifically binding complex through the porous filter membrane of any of claims 1 to 6, whereby the hapten binds the specifically binding complex to the membrane; and
   (c) detecting the label on the membrane.

10. An assay as claimed in any one of claims 7 to 9 wherein the substance to be determined is an antigen and the binding member to which the anti-hapten is bound is an antibody and the binding member which is labelled is an antibody.

11. An assay as claimed in claim 9 for an antibody comprising the steps of:
   (a) forming in solution a specifically binding complex between the antibody to be detected, a detectably labelled antigen to the antibody, a haptenated antigen to the antibody, and an antihapten;
   (b) filtering the solution containing the specifically binding complex through the porous filter membrane of any of claims 1 to 6; and
   (c) detecting the label attached on the membrane.

12. An assay as claimed in claim 9 for total DNA comprising the steps of:
   (a) denaturing the DNA to be detected to single-stranded DNA;
   (b) forming in solution a specifically binding complex between the single-stranded DNA to be detected, labelled anti-DNA or labelled SSB, which specifically binds to the single-stranded DNA to be detected,

haptenated anti-DNA or SSB which specifically binds to the DNA to be detected and anti-hapten;
(c) filtering the solution through the porous filter membrane of any of claims 1 to 6; and
(d) detecting the labelled anti-DNA or labelled SSB attached on the membrane.

**13.** An assay as claimed in claim 9 for rare RNA or rare DNA comprising the steps of:
(a) denaturing the rare RNA or DNA to be detected to single-stranded RNA or DNA;
(b) forming in solution a specifically binding complex between the single-stranded RNA or DNA to be detected, labelled oligonucleotide probe, which binds in a sequence-specific fashion to the single-stranded RNA or DNA to be detected, haptenated oligonucleotide probe which specifically binds to the RNA or DNA to be detected and anti-hapten;
(c) filtering the solution through the porous filter membrane of any of claims 1 to 6; and
(d) detecting the labelled oligonucleotide probe attached on the membrane.

**14.** An assay comprising the steps of:
(a) forming in solution a specifically binding complex between binding members and the substance to be determined wherein one binding member has bound to it a hapten and another binding member having a detectable label;
(b) filtering a solution containing an anti-hapten through the porous filter membrane of any of claims 1 to 6, whereby binding between the hapten and the anti-hapten captures the anti-hapten on the membrane;
(c) filtering the solution containing the specifically binding complex through the filter membrane having anti-hapten bound thereto, whereby binding between the hapten in the specifically binding complex and anti-hapten captures the specific binding complex on the membrane;
(d) detecting the label attached on the membrane.

**15.** A sequential assay which comprises the steps of:
(a) filtering a solution containing an anti-hapten through the porous filter membrane of any of claims 1 to 6, whereby binding between the hapten and the anti-hapten captures the anti-hapten on the membrane;
(b) filtering a solution containing an anti-hapten binding substance provided with binding sites for a substance to be determined through the filter membrane whereby the anti-hapten binding substance is captured on the membrane;
(c) filtering a solution containing or suspected to contain a substance to be determined through the membrane, whereby the substance to be determined is captured on the membrane;
(d) labelling the substance to be determined with a detectable label; and
(e) detecting the label.

**16.** An assay as claimed in claim 13 being a quantitative polymerase chain reaction assay characterised in that it comprises the steps of:
(a) removing a plurality of samples of amplified DNA from a polymerase chain reaction at various times;
(b) denaturing said amplified DNA;
(c) forming in solution a specifically binding complex between the amplified DNA to be detected in each sample, labelled oligonucleotide probe, which specifically binds to the amplified DNA to be detected, haptenated oligonucleotide probe which specifically binds to the amplified DNA to be detected and anti-hapten;
(d) filtering the solution through the porous filter membrane of any of claims 1 to 6;
(e) detecting the labelled oligonucleotide probe from each sample attached on the membrane;
(f) determining the amount of DNA formed by the polymerase chain reaction in each sample;
(g) extrapolating from the amounts of DNA in the respective samples the quantity of DNA present prior to amplification.

**17.** An assay as claimed in any one of claims 7 to 16 wherein the hapten is covalently bound to the macromolecule via an extended linking group.

**18.** An assay as claimed in any one of claims 7 to 17 wherein the macromolecule is a protein.

**19.** An assay as claimed in any one of claims 7 to 18 wherein the hapten is biotin and the anti-hapten is avidin or streptavidin.

20. An assay as claimed in any one of claims 7 to 10 and 12 to 19 wherein the binding members are antibodies which bind to the substance to be determined.

21. An assay as claimed in any one of claims 7 to 20 wherein the porous membrane is nitrocellulose, cellulose acetate or polyvinylidene difluoride polymer coated with a hydrophilic polymer.

22. An assay as claimed in any one of claims 7 to 21 wherein the label is an enzyme.

23. An assay as claimed in claim 22 wherein the enzyme is urease, horseradish peroxidase or alkaline phosphatase.

24. An assay as claimed in any one of claims 22 or 23 wherein the enzyme is detected by an electrode.

25. An assay as claimed in claim 24 wherein the electrode is a semiconductor.

26. An assay as claimed in claim 9 or claim 11 wherein the hapten is bound to the binding member with an extended linking group.

## Patentansprüche

1. Abfangmembran, umfassend eine poröse Filtermembran mit einem Hapten, das über ein an der Membran befestigtes und kovalent an das Hapten gebundenes Makromolekül indirekt an die Membran gebunden ist.

2. Abfangmembran gemäß Anspruch 1, wobei das Hapten über eine ausgedehnte Verknüpfungsgruppe kovalent an das Makromolekül gebunden ist.

3. Abfangmembran gemäß Anspruch 1 oder 2, wobei das Makromolekül ein Protein ist.

4. Abfangmembran gemäß einem der Ansprüche 1 bis 3, wobei das Hapten Biotin ist und das Antihapten Avidin oder Streptavidin ist.

5. Abfangmembran gemäß Anspruch 3, wobei das Protein Rinderserumalbumin, Rinder-$\gamma$-Albumin oder Fibrinogen ist.

6. Abfangmembran gemäß Anspruch 4 oder 5, wobei an jedes Rinderserumalbumin-Molekül etwa 5 bis 20 Biotinmoleküle gebunden sind.

7. Assay, bei dem ein spezifisch bindender Komplex durch eine Membran, die den spezifisch bindenden Komplex bindet, aus einer Lösung entfernt wird, woraufhin der Komplex an der Membran anhand einer nachweisbaren Markierung, die an den Komplex gebunden ist, nachgewiesen wird, dadurch gekennzeichnet, daß der Assay die Verwendung der porösen Filtermembran nach einem der Ansprüche 1 bis 6 zum Abfangen des spezifisch bindenden Komplexes durch Reaktion mit einem an ein Glied des spezifisch bindenden Komplexes gebundenen Antihapten umfaßt.

8. Assay gemäß Anspruch 7, der die folgenden Schritte umfaßt:
(a) Bilden eines spezifisch bindenden Komplexes zwischen bindenden Gliedern und der zu bestimmenden Substanz in Lösung, wobei eines der bindenden Glieder ein gebundenes Antihapten aufweist und ein anderes bindendes Glied eine nachweisbare Markierung aufweist;
(b) Filtrieren der Lösung, die den spezifisch bindenden Komplex enthält, durch die poröse Filtermembran nach einem der Ansprüche 1 bis 6, wobei der spezifisch bindende Komplex durch Binden zwischen dem Hapten und dem Antihapten auf der Membran abgefangen wird; und
(c) Nachweisen der an die Membran gebundenen Markierung.

9. Assay, der die folgenden Schritte umfaßt:
(a) Bilden eines spezifisch bindenden Komplexes zwischen der nachzuweisenden Substanz und bindenden Gliedern und einem Antihapten in Lösung, wobei eines der bindenden Glieder eine nachweisbare Markierung auf-weist und eines der bindenden Glieder ein gebundenes Hapten aufweist;
(b) Filtrieren der Lösung, die den spezifisch bindenden Komplex enthält, durch die poröse Filtermembran nach einem der Ansprüche 1 bis 6, wodurch das Hapten den spezifisch bindenden Komplex an die Membran bindet; und
(c) Nachweisen der Markierung an der Membran.

**10.** Assay gemäß einem der Ansprüche 7 bis 9, wobei die zu bestimmende Substanz ein Antigen ist und das bindende Glied, an das das Antihapten gebunden ist, ein Antikörper ist und das markierte bindende Glied ein Antikörper ist.

**11.** Assay gemäß Anspruch 9 für einen Antikörper, umfassend die folgenden Schritte:
(a) Bilden eines spezifisch bindenden Komplexes zwischen dem nachzuweisenden Antikörper, einem nachweisbar markierten Antigen für den Antikörper, einem mit einem Hapten versehenen Antigen für den Antikörper und einem Antihapten in Lösung;
(b) Filtrieren der Lösung, die den spezifisch bindenden Komplex enthält, durch die poröse Filtermembran nach einem der Ansprüche 1 bis 6; und
(c) Nachweisen der an die Membran gebundenen Markierung.

**12.** Assay gemäß Anspruch 9 für Gesamt-DNA, umfassend die folgenden Schritte:
(a) Denaturieren der nachzuweisenden DNA zu einzelsträngiger DNA;
(b) Bilden eines spezifisch bindenden Komplexes zwischen der nachzuweisenden einzelsträngigen DNA, markiertem Anti-DNA oder markiertem SSB, das spezifisch an die nachzuweisende einzelsträngige DNA bindet, mit einem Hapten versehenem Anti-DNA oder SSB, das spezifisch an die nachzuweisende einzelsträngige DNA bindet, und einem Antihapten in Lösung;
(c) Filtrieren der Lösung durch die poröse Filtermembran nach einem der Ansprüche 1 bis 6; und
(d) Nachweisen des an die Membran gebundenen markierten Anti-DNA oder markierten SSB.

**13.** Assay gemäß Anspruch 9 für seltene RNA oder seltene DNA, umfassend die folgenden Schritte:
(a) Denaturieren der nachzuweisenden seltenen RNA oder DNA zu einzelsträngiger RNA oder DNA;
(b) Bilden eines spezifisch bindenden Komplexes zwischen der nachzuweisenden einzelsträngigen RNA oder DNA, einer markierten Oligonucleotid-Sonde, die sequenzspezifisch an die nachzuweisende einzelsträngige RNA oder DNA bindet, mit einem Hapten versehener Oligonucleotid-Sonde, die spezifisch an die nachzuweisende RNA oder DNA bindet, und einem Antihapten in Lösung;
(c) Filtrieren der Lösung durch die poröse Filtermembran nach einem der Ansprüche 1 bis 6; und
(d) Nachweisen der an die Membran gebundenen markierten Oligonucleotid-Sonde.

**14.** Assay, der die folgenden Schritte umfaßt:
(a) Bilden eines spezifisch bindenden Komplexes zwischen bindenden Gliedern und der zu bestimmenden Substanz in Lösung, wobei eines der bindenden Glieder ein gebundenes Hapten aufweist und ein anderes bindendes Glied eine nachweisbare Markierung aufweist;
(b) Filtrieren einer Lösung, die ein Antihapten enthält, durch die poröse Filtermembran nach einem der Ansprüche 1 bis 6, wobei das Antihapten durch Binden zwischen dem Hapten und dem Antihapten auf der Membran abgefangen wird;
(c) Filtrieren der Lösung, die den spezifisch bindenden Komplex enthält, durch die Filtermembran mit daran gebundenem Antihapten, wobei der spezifisch bindende Komplex durch Binden zwischen dem Hapten in dem spezifisch bindenden Komplex und dem Antihapten auf der Membran abgefangen wird;
(d) Nachweisen der an die Membran gebundenen Markierung.

**15.** Sequentieller Assay, der die folgenden Schritte umfaßt:
(a) Filtrieren einer Lösung, die ein Antihapten enthält, durch die poröse Filtermembran nach einem der Ansprüche 1 bis 6, wobei das Antihapten durch Binden zwischen dem Hapten und dem Antihapten auf der Membran abgefangen wird;
(b) Filtrieren einer Lösung, die eine antihaptenbindende Substanz enthält, welche mit bindenden Bereichen für eine zu bestimmende Substanz versehen ist, durch die Filtermembran, wodurch die antihaptenbindende Substanz auf der Membran abgefangen wird;
(c) Filtrieren einer Lösung, die eine zu bestimmende Substanz enthält oder von der man dies annimmt, durch die Membran, wodurch die zu bestimmende Substanz auf der Membran abgefangen wird;
(d) Markieren der zu bestimmenden Substanz mit einer nachweisbaren Markierung; und

(e) Nachweisen der Markierung.

16. Assay gemäß Anspruch 13, bei dem es sich um einen quantita tiven Polymerase-Kettenreaktion-Assay handelt, dadurch gekennzeichnet, daß er die folgenden Schritte umfaßt:
(a) Entnehmen einer Mehrzahl von Proben amplifizierter DNA aus einer Polymerase-Kettenreaktion zu verschiedenen Zeiten;
(b) Denaturieren der amplifizierten DNA;
(c) Bilden eines spezifisch bindenden Komplexes zwischen der nachzuweisenden amplifizierten DNA in jeder Probe, einer markierten Oligonucleotid-Sonde, die spezifisch an die nachzuweisende amplifizierte DNA bindet, mit einem Hapten versehener Oligonucleotid-Sonde, die spezifisch an die nachzuweisende amplifizierte DNA bindet, und einem Antihapten in Lösung;
(d) Filtrieren der Lösung durch die poröse Filtermembran nach einem der Ansprüche 1 bis 6;
(e) Nachweisen der an die Membran gebundenen markierten Oligonucleotid-Sonde von jeder Probe;
(f) Bestimmen der Menge der durch die Polymerase-Kettenreaktion gebildeten DNA in jeder Probe;
(g) Extrapolieren der Menge der vor der Amplifikation vorhandenen DNA aus den Mengen der DNA in den jeweiligen Proben.

17. Assay gemäß einem der Ansprüche 7 bis 16, wobei das Hapten über eine ausgedehnte Verknüpfungsgruppe kovalent an das Makromolekül gebunden ist.

18. Assay gemäß einem der Ansprüche 7 bis 17, wobei das Makromolekül ein Protein ist.

19. Assay gemäß einem der Ansprüche 7 bis 18, wobei das Hapten Biotin ist und das Antihapten Avidin oder Streptavidin ist.

20. Assay gemäß einem der Ansprüche 7 bis 10 und 12 bis 19, wobei die bindenden Glieder Antikörper sind, die an die zu bestimmende Substanz binden.

21. Assay gemäß einem der Ansprüche 7 bis 20, wobei es sich bei der porösen Membran um Nitrocellulose, Celluloseacetat oder Polyvinylidendifluorid-Polymer, das mit einem hydrophilen Polymer beschichtet ist, handelt.

22. Assay gemäß einem der Ansprüche 7 bis 21, wobei die Markierung ein Enzym ist.

23. Assay gemäß Anspruch 22, wobei es sich bei dem Enzym um Urease, Meerrettich-Peroxidase oder Alkalische Phosphatase handelt.

24. Assay gemäß einem der Ansprüche 22 oder 23, wobei das Enzym durch eine Elektrode nachgewiesen wird.

25. Assay gemäß Anspruch 24, wobei es sich bei der Elektrode um einen Halbleiter handelt.

26. Assay gemäß Anspruch 9 oder 11, wobei das Hapten über eine ausgedehnte Verknüpfungsgruppe an das bindende Glied gebunden ist.

## Revendications

1. Membrane de capture comprenant une membrane filtrante poreuse comportant un haptène lié indirectement à la membrane par l'intermédiaire d'une macromolécule fixée à la membrane et liée par une liaison covalente à l'haptène.

2. Membrane de capture selon la revendication 1, dans laquelle l'haptène est lié par une liaison covalente à la macromolécule par l'intermédiaire d'un groupe liant allongé.

3. Membrane de capture selon la revendication 1 ou la revendication 2, dans laquelle la macromolécule est une protéine.

**4.** Membrane de capture selon l'une des revendications 1 à 3, dans laquelle l'haptène est la biotine et l'anti-haptène est l'avidine ou la streptavidine.

**5.** Membrane de capture selon la revendication 3, dans laquelle la protéine est la sérum albumine bovine, la gamma globuline bovine ou le fibrinogène.

**6.** Membrane de capture selon la revendication 4 ou la revendication 5, dans laquelle environ 5 à 20 molécules de biotine sont liées à chaque molécule de sérum albumine bovine.

**7.** Dosage selon lequel un complexe liant spécifique est éliminé d'une solution à l'aide d'une membrane qui se lie au complexe liant spécifique, après quoi ledit complexe sur ladite membrane est détecté au moyen d'un marqueur détectable fixé audit complexe, dosage caractérisé en ce qu'il comprend l'utilisation de la membrane filtrante poreuse selon l'une des revendications 1 à 6, dans le but de retenir ledit complexe liant spécifique par réaction avec un anti-haptène lié à une partie du complexe liant spécifique.

**8.** Dosage selon la revendication 7 comprenant les étapes suivantes:
(a) formation en solution d'un complexe liant spécifique entre les parties liantes et la substance à déterminer, dans lequel une partie liante est liée à un anti-haptène et à une autre partie liante comportant un marqueur détectable;
(b) filtration de la solution contenant le complexe liant spécifique sur la membrane filtrante poreuse selon l'une des revendications 1 à 6, la liaison entre l'haptène et l'anti-haptène retenant ainsi le complexe liant spécifique sur la membrane; et
(c) détection du marqueur fixé à la membrane.

**9.** Dosage comprenant les étapes suivantes:
(a) formation en solution d'un complexe liant spécifique entre la substance à déterminer et les parties liantes et un anti-haptène, dans lequel une partie liante comporte un marqueur détectable, et une autre partie liante est liée à un haptène;
(b) filtration de la solution contenant le complexe liant spécifique sur la membrane filtrante poreuse selon l'une des revendications 1 à 6, au cours de laquelle l'haptène lie le complexe liant spécifique à la membrane; et
(c) détection du marqueur fixé à la membrane.

**10.** Dosage selon l'une des revendications 7 à 9, dans lequel la substance à déterminer est un antigène et la partie liante à laquelle l'anti-haptène est lié est un anticorps, et la partie liante qui est marquée est un anticorps.

**11.** Dosage d'un anticorps selon la revendication 9, pour doser un anticorps comprenant les étapes suivantes:
(a) formation en solution d'un complexe liant spécifique entre l'anticorps à détecter, un antigène marqué de façon détectable et spécifique de l'anticorps, un antigène lié à un haptène et spécifique de l'anticorps, et un anti-haptène;
(b) filtration de la solution contenant le complexe liant spécifique sur la membrane filtrante poreuse selon l'une des revendications 1 à 6; et
(c) détection du marqueur fixé à la membrane.

**12.** Dosage selon la revendication 9 pour doser de l'ADN total comprenant les étapes suivantes:
(a) dénaturation de l'ADN à détecter en ADN simple brin;
(b) formation en solution d'un complexe liant spécifique entre l'ADN simple brin à détecter, un anti-ADN marqué ou une protéine fixatrice d'ADN simple brin marquée, qui se lie spécifiquement à l'ADN simple brin à détecter, un anti-ADN ou une protéine fixatrice d'ADN simple brin lié à un haptène, qui se lie spécifiquement l'ADN à détecter, et un anti-haptène;
(c) filtration de la solution sur la membrane filtrante poreuse selon l'une des revendications 1 à 6; et
(d) détection de l'anti-ADN marqué ou de la protéine fixatrice d'ADN simple brin marquée fixé sur la membrane.

**13.** Dosage selon la revendication 9 pour doser un ARN rare ou d'un ADN rare comprenant les étapes suivantes:
(a) dénaturation de l'ARN rare ou de l'ADN rare à détecter, en ARN ou en ADN simple brin;

(b) formation en solution d'un complexe liant spécifique entre l'ARN ou l'ADN simple brin à détecter, une sonde oligonucléotidique marquée, qui se lie, grâce à sa séquence de manière spécifique, à l'ARN ou à l'ADN simple brin à détecter, une sonde oligonucléotidique conjuguée à un haptène, qui se lie spécifiquement à l'ARN ou à l'ADN à détecter, et un anti-haptène;
(c) filtration de la solution sur la membrane filtrante poreuse selon l'une des revendications 1 à 6; et
(d) détection de la sonde oligonucléotidique marquée fixée sur la membrane.

**14.** Dosage comprenant les étapes suivantes:
(a) formation en solution d'un complexe liant spécifique entre des parties liantes et la substance à déterminer, dans lequel une partie liante est liée à un haptène et une autre partie liante comportant un marqueur détectable;
(b) filtration d'une solution contenant un anti-haptène sur la membrane filtrante poreuse selon l'une des revendications 1 à 6, au cours de laquelle la liaison entre l'haptène et l'anti-haptène retient l'anti-haptène sur la membrane;
(c) filtration de la solution contenant le complexe liant spécifique sur la membrane filtrante à laquelle est lié l'anti-haptène, au cours de laquelle la liaison entre l'haptène du complexe liant spécifique et l'anti-haptène retient le complexe liant spécifique sur la membrane;
(d) détection du marqueur fixé à la membrane.

**15.** Dosage séquentiel qui comprend les étapes suivantes:
(a) filtration d'une solution contenant un anti-haptène sur la membrane filtrante poreuse selon l'une des revendications 1 à 6, au cours de laquelle la liaison entre l'haptène et l'anti-haptène retient l'anti-haptène sur la membrane;
(b) filtration, sur la membrane filtrante, d'une solution contenant une substance se liant à l'anti-haptène comportant des sites de liaison pour une substance à déterminer, ce qui retient la substance se liant à l'anti-haptène sur la membrane;
(c) filtration d'une solution contenant ou suspectée de contenir une substance à déterminer sur la membrane, ce qui retient la substance à déterminer sur la membrane;
(d) marquage de la substance à déterminer par un marqueur détectable; et
(e) détection du marqueur.

**16.** Dosage selon la revendication 13 qui est un dosage quantitatif par réaction en chaîne de la polymérase caractérisé en ce qu'il comprend les étapes suivantes :
(a) récupération d'une pluralité d'échantillons d'ADN amplifié par une réaction en chaîne de la polymérase à différents moments;
(b) dénaturation dudit ADN amplifié;
(c) formation en solution d'un complexe liant spécifique entre l'ADN amplifié à détecter dans chaque échantillon, une sonde oligonucléotidique marquée, qui se lie spécifiquement à l'ADN amplifié à détecter, une sonde oligonucléotidique conjuguée à un haptène qui se lie spécifiquement à l'ADN amplifié à détecter, et un anti-haptène;
(d) filtration de la solution sur la membrane filtrante poreuse selon l'une des revendications 1 à 6;
(e) détection de la sonde oligonucléotidique marquée provenant de chaque échantillon et fixée à la membrane;
(f) détermination de la quantité d'ADN formé par la réaction en chaîne de la polymérase dans chaque échantillon;
(g) détermination, par extrapolation à partir des quantités d'ADN de chaque échantillon, de la quantité d'ADN présente avant l'amplification.

**17.** Dosage selon l'une des revendications 7 à 16, selon lequel l'haptène est lié par une liaison covalente à la macromolécule par l'intermédiaire d'un groupe liant allongé.

**18.** Dosage selon l'une des revendications 7 à 17, selon lequel la macromolécule est une protéine.

**19.** Dosage selon l'une des revendications 7 à 18, selon lequel l'haptène est la biotine et l'anti-haptène est l'avidine ou la streptavidine.

**20.** Dosage selon l'une des revendications 7 à 10 et 12 à 19, selon lequel les parties liantes sont des anticorps qui se lient à la substance à déterminer.

**21.** Dosage selon l'une des revendications 7 à 20, selon lequel la membrane poreuse est un polymère de nitrocellulose, d'acétate de cellulose ou de difluorure de polyvinylidène revêtu d'un polymère hydrophile.

**22.** Dosage selon l'une des revendications 7 à 21, selon lequel le marqueur est une enzyme.

**23.** Dosage selon la revendication 22, selon lequel l'enzyme est l'uréase, la peroxydase de raifort ou la phosphatase alcaline.

**24.** Dosage selon la revendication 22 ou 23, selon lequel l'enzyme est détectée par une électrode.

**25.** Dosage selon la revendication 24, selon lequel l'électrode est un semiconducteur.

**26.** Dosage selon la revendication 9 ou la revendication 11, selon lequel l'haptène est lié à la partie liante par un groupe de couplage allongé.

Fig.1

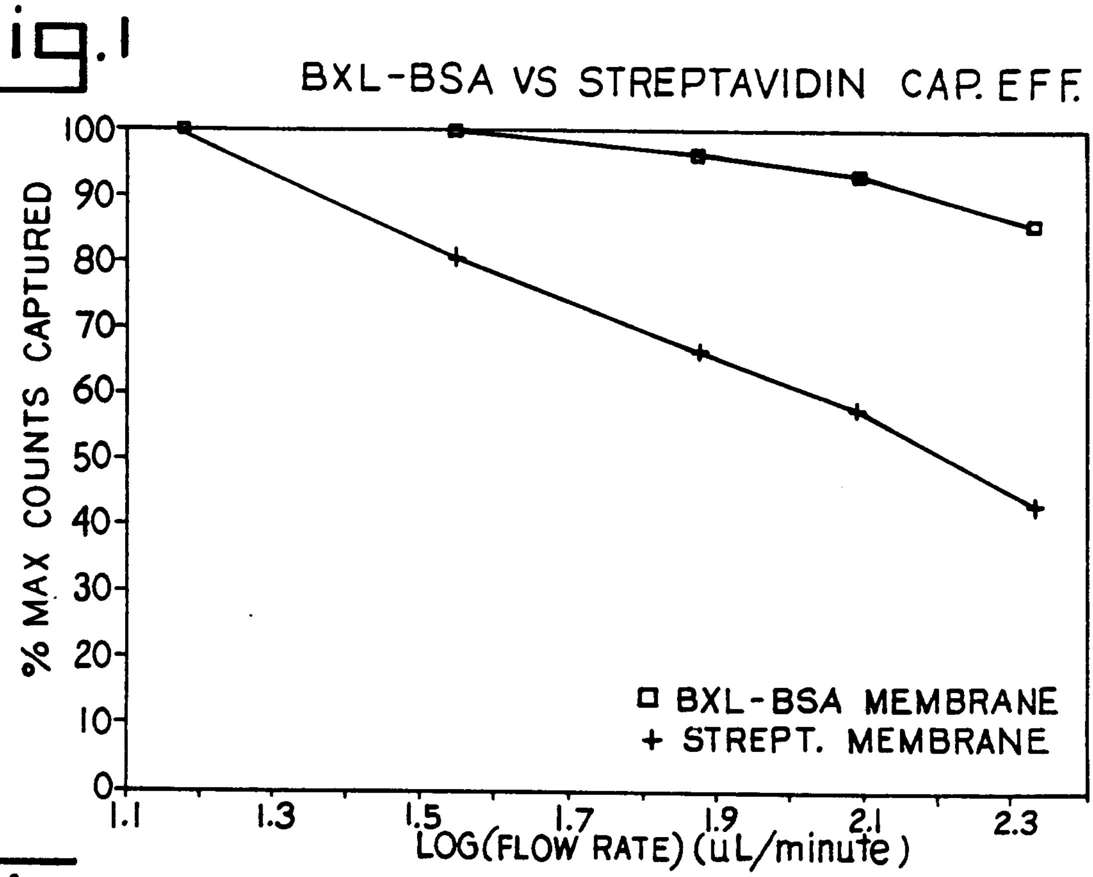
BXL-BSA VS STREPTAVIDIN CAP. EFF.
% MAX COUNTS CAPTURED
100
90
80
70
60
50
40
30
20
10
0
1.1
1.3
1.5
1.7
1.9
2.1
2.3
LOG(FLOW RATE) (uL/minute)
□ BXL-BSA MEMBRANE
+ STREPT. MEMBRANE

Fig.2

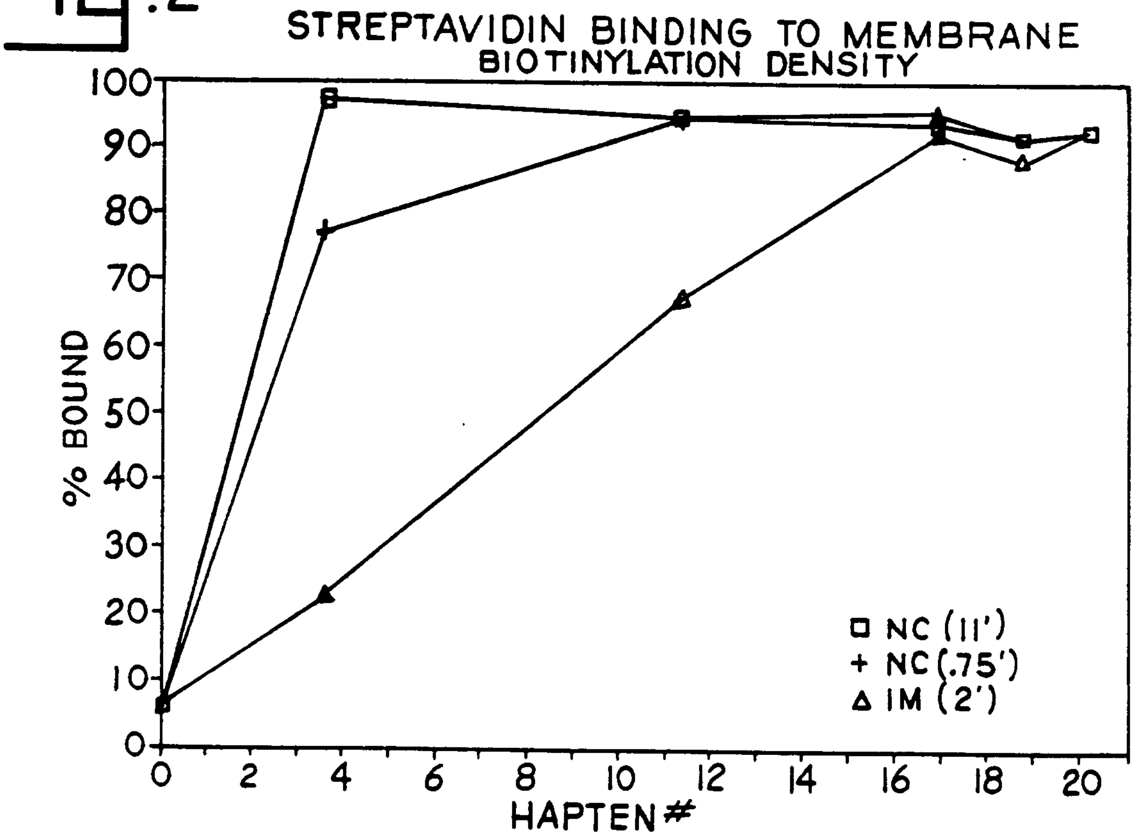
STREPTAVIDIN BINDING TO MEMBRANE
BIOTINYLATION DENSITY
% BOUND
100
90
80
70
60
50
40
30
20
10
0
0
2
4
6
8
10
12
14
16
18
20
HAPTEN #
□ NC (11')
+ NC (.75')
Δ IM (2')

Fig.3

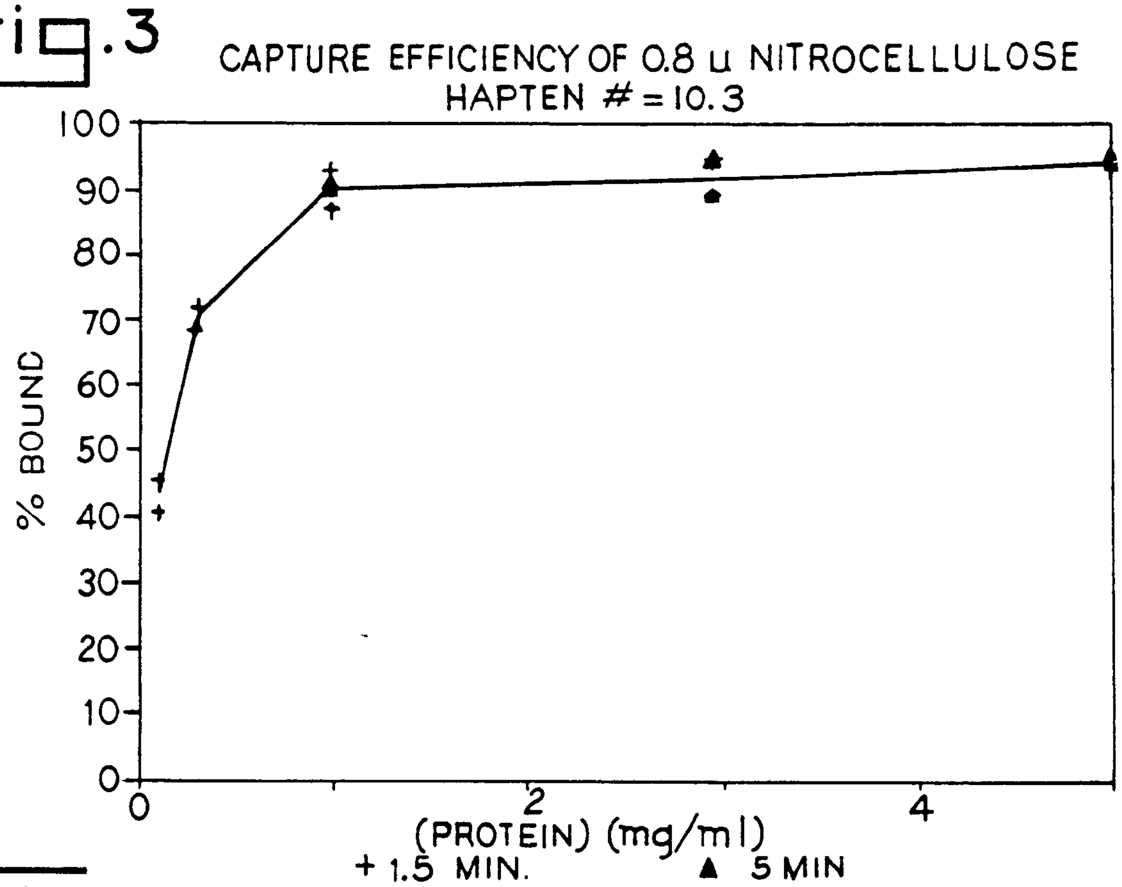
CAPTURE EFFICIENCY OF 0.8 u NITROCELLULOSE
HAPTEN # = 10.3
% BOUND
100
90
80
70
60
50
40
30
20
10
0
2
4
(PROTEIN) (mg/ml)
+ 1.5 MIN.
▲ 5 MIN

Fig.4

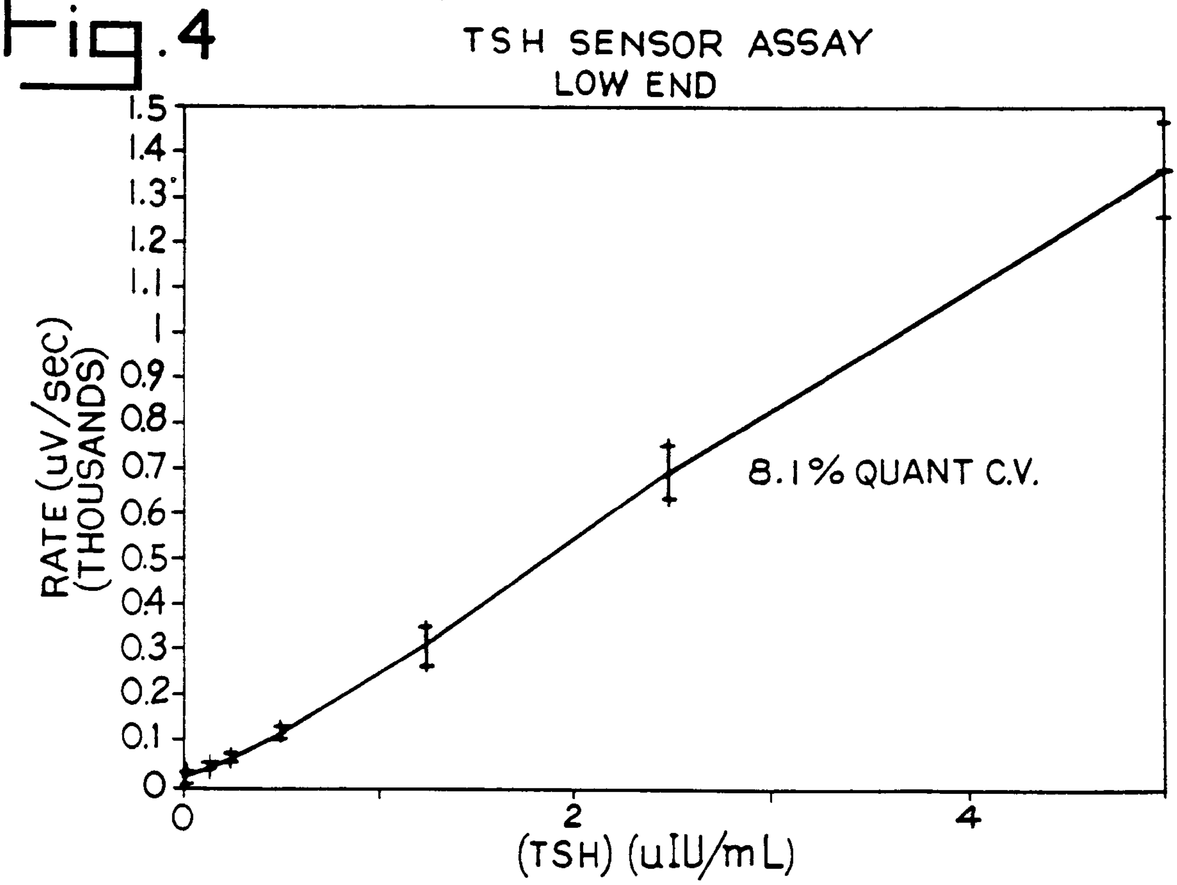
TSH SENSOR ASSAY
LOW END
RATE (uV/sec)
(THOUSANDS)
1.5
1.4
1.3
1.2
1.1
1
0.9
0.8
0.7
0.6
0.5
0.4
0.3
0.2
0.1
0
2
4
(TSH) (uIU/mL)
8.1% QUANT C.V.

Fig. 5

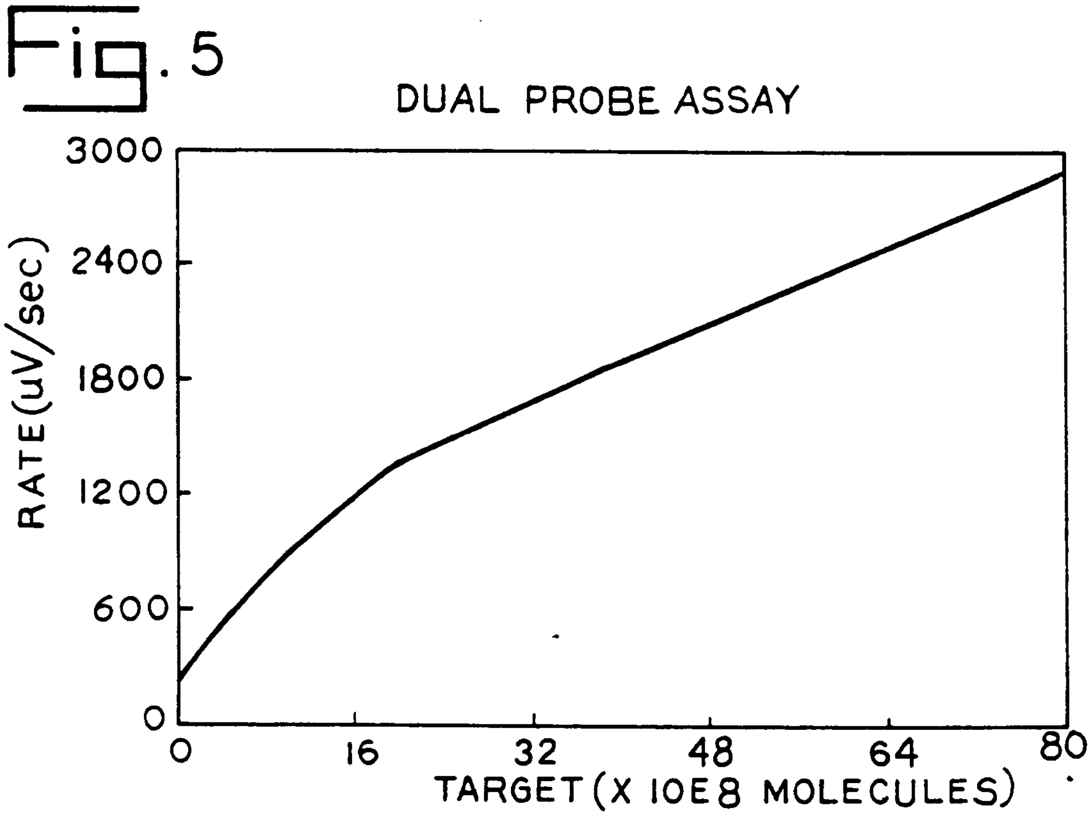
DUAL PROBE ASSAY
RATE (uV/sec)
3000
2400
1800
1200
600
0
0
16
32
48
64
80
TARGET (X 10E8 MOLECULES)

Fig. 6

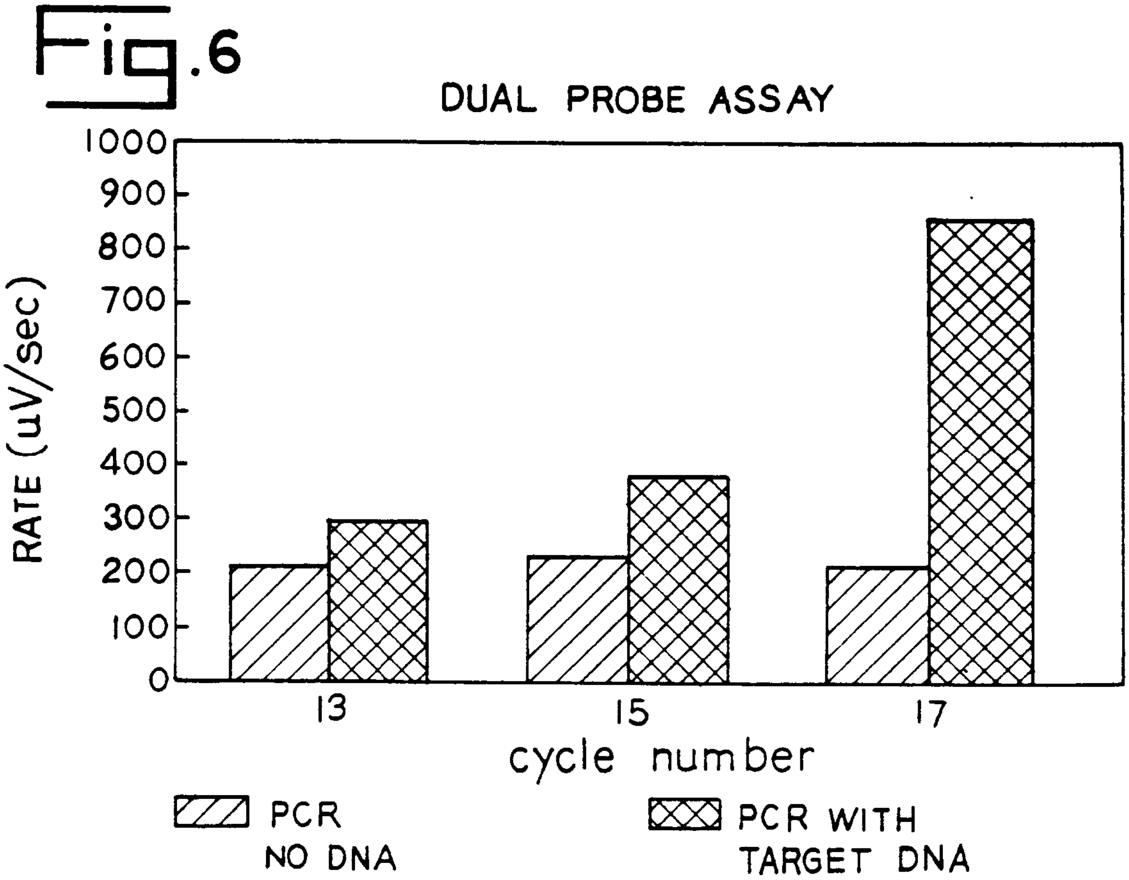
DUAL PROBE ASSAY
RATE (uV/sec)
1000
900
800
700
600
500
400
300
200
100
0
13
15
17
cycle number
PCR
NO DNA
PCR WITH
TARGET DNA

Fig. 7

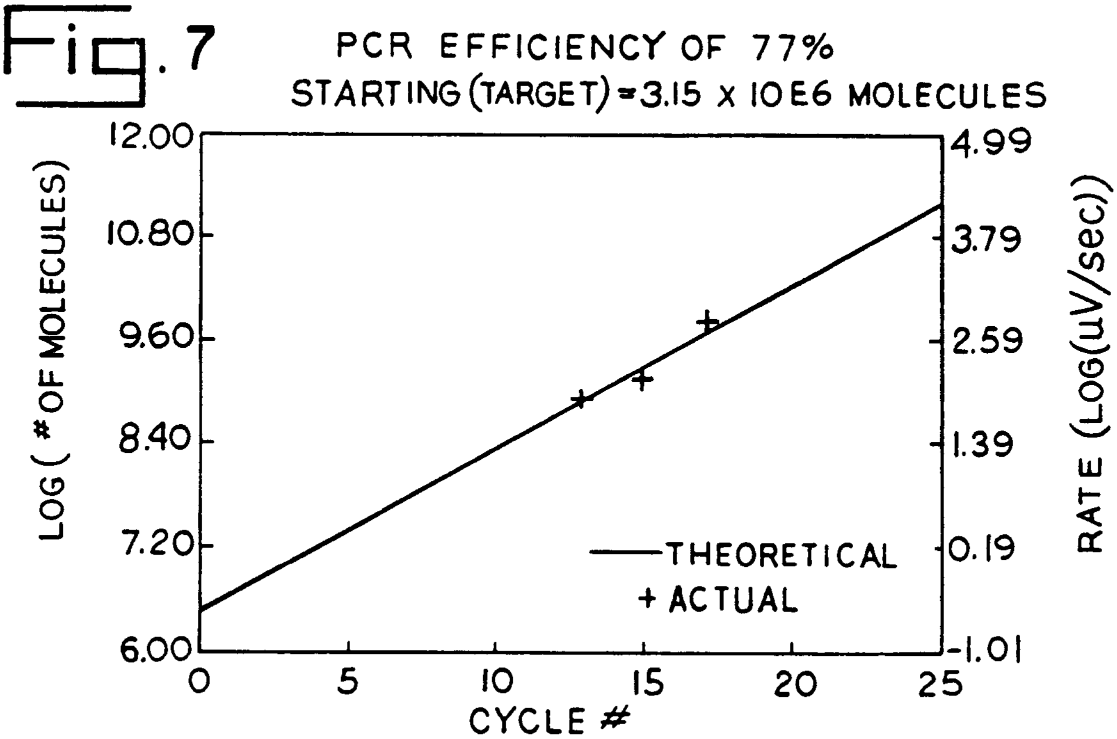
PCR EFFICIENCY OF 77%
STARTING (TARGET) = 3.15 x 10E6 MOLECULES
LOG ( # OF MOLECULES)
12.00
10.80
9.60
8.40
7.20
6.00
RATE (LOG(uV/sec))
4.99
3.79
2.59
1.39
0.19
-1.01
0
5
10
15
20
25
CYCLE #
THEORETICAL
+ ACTUAL

Fig. 8

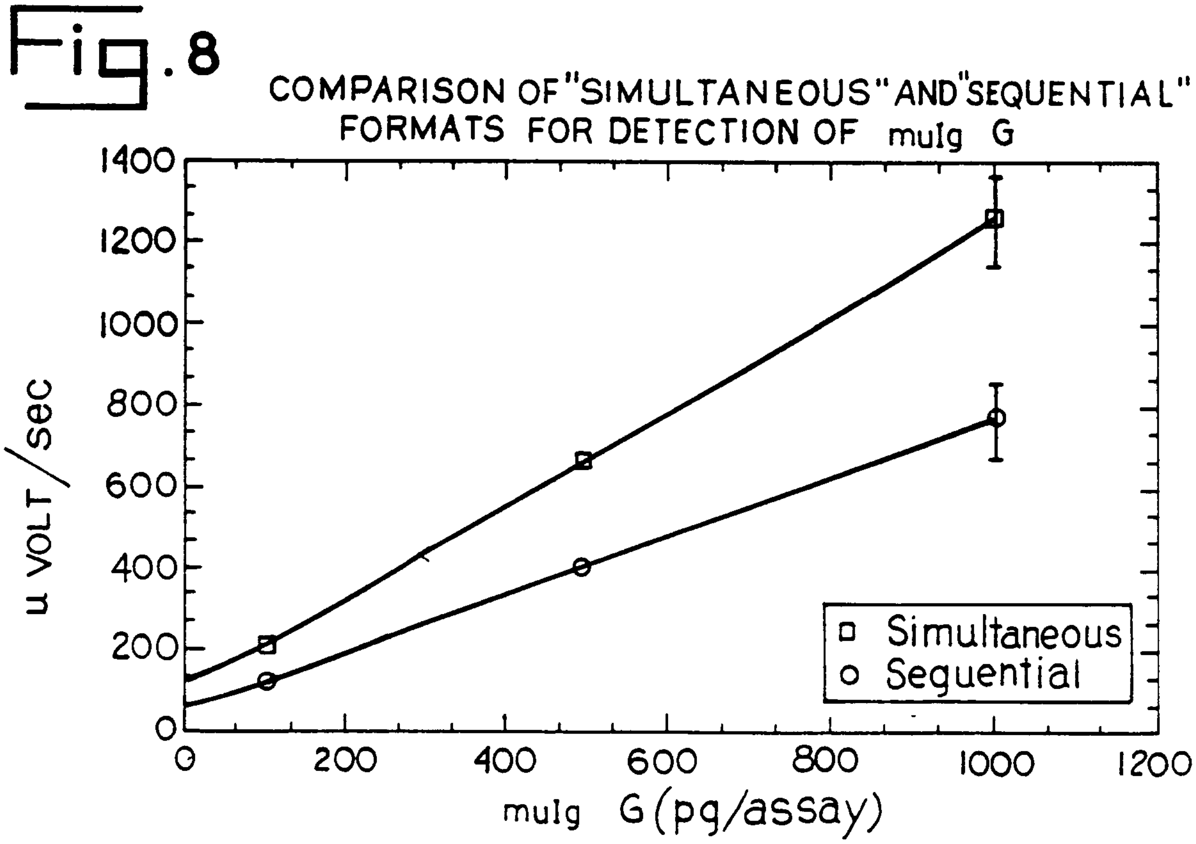
COMPARISON OF "SIMULTANEOUS" AND "SEQUENTIAL" FORMATS FOR DETECTION OF muIg G
u VOLT/sec
1400
1200
1000
800
600
400
200
0
0
200
400
600
800
1000
1200
muIg G (pg/assay)
Simultaneous
Sequential